# EUROPÄISCHE PATENTSCHRIFT

(11) **EP 3 389 621 B1**
(45) Veröffentlichungstag und Bekanntmachung des Hinweises auf die Patenterteilung: **19.10.2022**
(21) Anmeldenummer: 16816649.4
(22) Anmeldetag: 15.12.2016
(51) Int. Cl.: A61K 8/64, A61Q 17/04, A61K 8/06

(54) **EMULSIONEN MIT UV-LICHTSCHUTZFAKTOREN UND CARNOSINEN**
EMULSIONS CONTAINIG UV-SCREENS AND CARNOSINES
ÉMULSIONS CONTENANT DES FILTRES UV ET DES CARNOSINES

(30) Priorität: 15.12.2015 WO PCT/EP2015/079711
(43) Veröffentlichungstag der Anmeldung: 24.10.2018
(73) Patentinhaber: Symrise AG, 37603 Holzminden (DE)
(72) Erfinder: VIELHABER, Gabriele, 92700 Colombes (FR)
(74) Vertreter: Fabry, Bernd
(86) Internationale Anmeldenummer: PCT/EP2016/081238
(87) Internationale Veröffentlichungsnummer: WO 2017/102971

(56) Entgegenhaltungen:
- EP-A2- 2 181 697
- EP-B1- 2 181 697
- WO-A2-2014/128228
- US-A1- 2009 258 964
- US-A1- 2013 116 342

## Beschreibung

### GEBIET DER ERFINDUNG

Die Erfindung befindet sich auf dem Gebiet der Kosmetik und betrifft speziell Sonnenschutzmittel in Emulsionsform mit verbesserter Lagerstabilität.

### STAND DER TECHNIK

Einer Statistik aus dem Jahre 2015 nach liegt die Zahl der Personen über 14 Jahre in Deutschland, die an mehr als 20 Tagen im Jahr Sonnenschutzprodukte anwenden, bei etwa 20 Millionen und ist über die letzten Jahre ständig im Anstieg begriffen. Damit zählen Sonnenschutzmittel zu den meistgefragtesten Kosmetikprodukten, zumal sich nach aktuellen Studien die Zahl der durch UV-Strahlung verursachten Hautkrebserkrankungen seit dem Jahre 2003 mehr als verdoppelt hat.

Sonnenschutzmittel sind hinsichtlich Zusammensetzung, Qualität und damit auch letztlich Preis sehr verschieden. Einfache Rezepturen, die vor allem von Discountern angeboten werden, sind einfach aufgebaut und enthalten nur wenige Lichtschutzfaktoren in geringen Mengen, was allenfalls bei vorgebräunter Haut zu einem Kurzzeitschutz führt. Andere Produkte weisen einen "Cocktail" von Lichtschutzfaktoren auf, d.h. hier wird ausgenutzt, dass Lichtschutzfaktoren ihr Absorptionsmaximum bei unterschiedlichen Wellenlängenbereichen haben und bei intelligenter Auswahl so ein gleichmäßiger Schutz über ein breites Spektrum des weißen Sonnenlichtes erzielt wird. Es liegt auf der Hand, dass diese Produkte nicht nur qualitativ hochwertiger, sondern eben auch für den Verbraucher teurer sind.

Ungebrochen ist der Trend, kosmetische Mittel durch Zusatz von weiteren Wirkstoffen in ihrer Wirkung zu verbessern. Im Bereich der Sonnenschutzprodukte besteht beispielsweise großes Interesse daran, Wirkstoffe zu identifizieren, die den Schutzbereich erweitern, d.h. nicht nur Schutz gegen lichtinduzierte Hautschädigung oder Hautalterung (UV-Einfluss) zu gewähren, sondern auch gegenüber anderen Einflüssen, wie beispielsweise Umweltgifte. Tatsache ist nämlich, dass Lichtschutzfaktoren gegen umweltinduzierte Schädigungen der Haut weitgehend oder gar völlig nutzlos sind, da diese völlig anderen Reaktionswegen in der Zelle folgen.

Mit dem Einbringen neuer bzw. zusätzlicher Wirkstoffe ist jedoch nicht nur ein Zuwachs an Qualität verbunden, auch die Probleme wachsen. Nicht jeder Wirkstoff lässt sich in jede beliebige Formulierung einarbeiten, die Komplexität bei Herstellung und Lagerung steigt und unter Umständen muss auch noch darauf Rücksicht genommen werden, dass der Zusatzstoff selbst wieder empfindlich gegen Luft- oder Lichteinfluss ist.

Besonders interessant sind entsprechende Zubereitungen in Emulsionsform, bei denen die Tröpfchen einen besonders kleinen Durchmesser aufweisen, da sie sich nicht nur leicht versprühen lassen, sondern auch ein hohes Spreitvermögen aufweisen, d.h. rasch in die Haut einziehen. Derartige Emulsionen zeichnen sich dadurch aus, dass die Tröpfchen so klein sind, dass sie das Licht nicht streuen. Die Lösungen erscheinen daher transparent. Der Nachteil solcher Formulierungen besteht indes darin, dass sie bei längerer Lagerung und insbesondere bei hohen Temperaturen keine ausreichende Stabilität aufweisen und sich ebenso so leicht entmischen, wie sie sich gebildet haben. Gerade dies sind aber Bedingungen, denen gerade Sonnenschutzmittel regelmäßig unterliegen.

Grundsätzlich ist das für das Produkt unschädlich, da es sich durch mehrfaches kräftiges Schütteln wieder homogenisieren lässt. Für einen Verbraucher, der sich gerade für ein hochqualitatives und entsprechend teures Produkt entschieden hat, ist dies aber unbefriedigend und wird gegebenenfalls dazu führen, dass seine nächste Kaufentscheidung anders ausfällt.

Die Aufgabe der vorliegenden hat daher darin bestanden, Sonnenschutzmittel auf Emulsionsbasis zur Verfügung zu stellen, die auch bei längerer Temperaturlagerung hinreichend stabil sind. Teil dieser Aufgabe war es, entsprechende Stabilisatoren aufzufinden, die nach Möglichkeit nicht nur eine funktionelle Aufgabe erfüllen, sondern darüber hinaus auch noch einen Wirkstoffcharakter aufweisen, der für den Anwendungsbereich Sonnenschutz vorteilhaft ist.

### BESCHREIBUNG DER ERFINDUNG

Die Erfindung betrifft die Verwendung von Carnosinverbindungen der Formel (I) 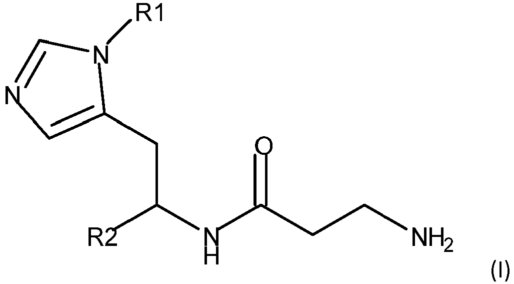 wobei R1 für H oder CH₃ und R2 für H oder COOH steht, oder deren Salze; als Stabilisatoren für Emulsionen.

Die Emulsionen können als O/W oder W/O-Emulsionen oder als multiple W/O/W bzw. O/W/O-Emulsionen vorliegen.

Überraschenderweise wurde mit den Carnosinen eine Gruppe von Stoffen aufgefunden, die nicht nur teils bekannte und teils bisher nicht bekannte Wirkstoffeigenschaften im Zusammenhang mit dem Schutz der Haut gegen licht- und umweltinduzierte Schädigungen aufweisen, sondern auch noch in der Lage sind, Emulsionen auf Basis von UV-A- und/oder UV-B-Lichtschutzfaktoren auch bei hohen Temperaturen und über längere Zeiträume lagerstabil zu halten. Damit erfüllen Carnosine das eingangs geschilderte Anforderungsprofil perfekt, indem sie den Formulierungen einen Wirkungseffekt verleihen und gleichzeitig noch eine funktionelle Aufgabe, nämlich als Stabilisator fungieren.

Die Stabilisierung von Emulsionen durch Peptide wird im US Patent US 2013/0116342 beschrieben. Carnosinderivate sind aber in diesem Dokument nicht offenbart.

### UV-LICHTSCHUTZFAKTOREN

Unter UV-Lichtschutzfaktoren (Komponente a) sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form langwelliger Strahlung, z.B. Wärme wieder abzugeben. Üblicherweise sind die UV-Lichtschutzfaktoren jeweils für sich betrachtet in Mengen von 0,1 bis 5 und vorzugsweise 0,2 bis 1 Gew.-% zugegen. Die Gesamtmenge an Lichtschutzfaktoren in einer Zubereitung kann indes deutlich höher liegen und je nach SPF ("sun protection factor") bis zu 25 Gew.-%, vorzugsweise etwa 10 bis etwa 15 Gew.-% betragen.

UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher beschrieben;
- 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)-benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-iso-propylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4methoxybenzophenon;
- Ester der Benzalmalonsäure, vorzugsweise 4-Methoxybenzmalonsäuredi-2-ethyl-hexylester;
- Triazinderivate, wie z.B. 2,4,6-Trianilino-(p-carbo-2'-ethyl-1'-hexyloxy)-1,3,5-triazin und Octyl Triazon oder Dioctyl Butamido Triazone (Uvasorb^{®} HEB);
- Propan-1,3-dione, wie z.B. 1-(4-tert.Butylphenyl)-3-(4'methoxyphenyl)propan-1,3-dion;
- Ketotricyclo(5.2.1.0)decan-Derivate.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- 1H-Benzimidazole-4,6-Disulfonic Acid, 2,2'-(1,4-Phenylene)Bis-, Disodium Salt (Neo Heliopan^{®} AP)
- Sulfonsäurederivate von Benzophenonen, vorzugsweise 2-Hydroxy-4-methoxybenzo-phenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxy-dibenzoylmethan (Parsol^{®} 1789), 2-(4-Diethylamino-2-hydroxybenzoyl)-benzoic acid hexylester (Uvinul^{®} A Plus), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans, z.B. 4-tert.-Butyl-4'-methoxydibenzoylmethan (Parsol^{®} 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäure-isoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

### CARNOSINE

Bei den Carnosinen, die die Formel (I) ausmachen und die Komponente (b) bilden, handelt es sich um grundsätzlich bekannte Verbindungen, die nach den üblichen Verfahren der organischen Chemie zugänglich sind. Vorzugsweise handelt es sich dabei um Gruppe von Stoffen, die gebildet wird von Carnosin, L-Carnosin, D-Carnosin, D/L-Carnosin, Carnicin, Carnicin^{∗}HCl, Anserin, D-Anserin, L-Anserin sowie L-Anserin^{∗}HNO₃ und deren Mischungen.

Unter Salzen der Verbindungen der Formel (I) werden erfindungsgemäß bevorzugt Salze der Verbindungen der Formel (I) mit mineralischen Säuren verstanden und besonders bevorzugt Salze der Formel (II): 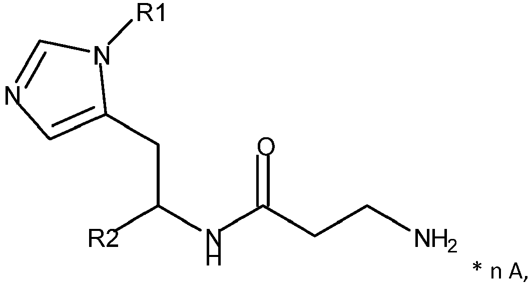 wobei n für 1,2 oder 3 und A für HCl oder HNO₃ steht und R1 für H oder CH₃ sowie R2 für H oder COOH.

Die vorteilhafte Verwendung von Carnosinen im Bereich der Prophylaxe gegen lichtinduzierte Hautalterung oder Hautschädigung ist aus dem Stand der Technik hinreichend bekannt.

So ist aus einer Untersuchung von *Aruoma et al.* schon aus dem Jahre 1989 beispielsweise bekannt, dass Carnosin in der Lage ist ROS *(*"reactive *oxygen species")* abzufangen [Biochem. J. 264(3), S. 863-869 (1989)]. Carnosin kann nach einem Bericht von Babizhayev et al. darüber hinaus alpha-beta-ungesättigte Aldehyde, die bei der Peroxidation von Fettsäuren der Zellmembran bei oxidativem Stress freigesetzt werden, abfangen [Membran & Cell Biol. 12(1), S. 89-99 (1998)].

**Die** EP 1310238 A2 (BASF) offenbart kosmetische oder dermatologische Lichtschutzmittelzubereitungen, die wenigstens eine im infraroten Wellenlängenbereich von 750 nm bis 2500 nm reflektierende cholesterisch-flüssigkristalline Komponente, wenigstens eine vor Strahlung im ultravioletten Strahlenbereich von 280 nm bis 449 nm schützende Filtersubstanz und wenigstens einen kosmetisch akzeptablen Träger enthalten sowie deren Verwendung. Darüber hinaus können weiter Antioxidantien, wie z.B. Carnosin zugegen sein.

**Die** EP 1388339 A1 (VISA) offenbart die Verwendung von Carnosin zur Kontrolle des Alterungsprozesses, indem die Faltenbildung der Haut umgekehrt, verhindert und reduziert wird. Carnosine wird als Anti-Falten Wirkstoff bezeichnet, der u.a. auch gegen Glycosylierung und chronische Entzündungen schützt.

Gegenstand der EP 2181697 A2 (SHISEIDO) sind Sonnenschutzmittel in O/W-Form, die sich durch einen speziellen Cocktail von Emulgatoren und Lichtschutzfiltern auszeichnen. Tabelle 3-3 offenbart zwei Zusammensetzungen (Beispiele 22 und 23), welche die Kombination von Carnosin mit UV-Filtern enthalten.

Darüber hinaus sind aus der EP 1591105 A1 (STADA) kosmetische und pharmazeutische Zubereitungen bekannt, die ein Antioxidantien enthalten, die die Haut vor IR-Strahlung schützen. Unter den zahlreichen möglichen Antioxidantien werden auch Carnosin und Arnesin genannt. Ferner umfassen die topisch zu verabreichenden Zusammensetzungen einen oder mehrere anorganische und/oder organische UV-Filter. Eine konkrete Offenbarung zu Mischungen von Carnosinen und UV-Filtern enthält diese Schrift nicht.

Die EP 2545898 A1 (COTY) offenbart Zubereitungen, die die Haut vor Schädigung durch IR-Strahlen schützen und dabei Pflanzenextrakte, Vitamine, Rubinpulver, Mica und Titandioxid enthalten. In Abschnitt [0026] wird als weiterer geeigneter Hilfsstoff auch Carnosin genannt, jedoch als Radikalfänger. Eine konkrete Offenbarung für Mischungen von Carnosinen und UV-Filtern enthält auch diese Schrift nicht.

Die internationale Patentanmeldung WO 1990 006102 A1 (PEPTIDE TECHNOLOGY) betrifft ebenfalls die anti-oxidative Eigenschaft von Carnosinverbindungen. Hauptsächlich wird offenbart, dass die gefundenen Verbindungen die Kollagenvernetzungen in der Haut reduziert oder verhindert und/oder die Schädigung der DNA Hautzellen, insbesondere verursacht durch Alterung oder UV Strahlung. Die Anwendung erfolgt topisch oder per Injektion. Ein Hinweis auf die Mischung mit UV-Filtern findet sich in der Schrift nicht.

Die WO 2010 124817 A2 (KPSS) betrifft Konditioniermittel auf Basis unter anderen von Dipeptiden wie beispielsweise Carnosin für die Haare. In den Ausführungsbeispielen findet sich eine Offenbarung für die Kombination von Carnosin mit einem UV-Filter, nämlich Benzophenon-3 in den Beispielen 4, 8, 9 und 16, wobei das Gewichtsverhältnis zwischen Carnosin und Benzophenon-3 im Bereich von 1:03 bis 1:2 liegt.

Weiterhin wird auf die internationale Patentanmeldung WO 2014 128228 A2 (SYM-RISE) verwiesen, die die Eignung dieser Stoffe zum Schutz gegen IR-Strahlung an Hand der Inhibierung der MMP-Expression aufzeigt. Konkret werden hier gewichtsgleiche Mischungen von Carnosin mit Uvinul A und Octocrylen offenbart.

Gegenstand der US 2006/216251 A1 (TRACIE MARTIN) sind topische Zusammensetzungen, enthaltend Carnosin, Carnitin und eine Liponsäure, zur Behandlung und Schutz der Haut, insbesondere bezüglich Anzeichen von Hautalterung in Bezug auf Chrono-Ageing oder Photo-Ageing, welche auf Falten, Hautschwächung, und nachlassen der Elastizität gerichtet ist. Die Behandlung gerichtet auf Hyperpigmentierung wird ebenfalls offenbart. In der Schrift wird ebenfalls auf den Einfluss von Carnosinen auf die MMP-1 Inhibierung hingewiesen. Die Beispiele 1 bis 8 offenbaren eine Einsatzmenge von Carnosin im Bereich 1 Gew.-% und 2 Gew.-%.

Gemäß der englischsprachigen Zusammenfassung der KR 2004 0074795 A (A E KYUNG) weisen 0.1 bis 2 Gew.-% Carnosin in kosmetischen Zusammensetzungen einen antioxidativen Effekt auf die Haut auf und erhöhen die Kollagensynthese. Als Beispiel wird eine Formulierung enthaltend 0.1 Gew.-% Carcinine (Carnosin), 0.5 Gew.-% Salicylsäure, 5.0 Gew.- % 1,3-Butylenglykol, 1.3 Gew.-% Polyethylensorbitanmonolaurat, 0.7 Gew.-% Polyethylenlaurylether, 15 Gew.-% Ethanol, 0.2 Gew.-% Duftstoffe, Konservierungsstoffe und Wasser angegeben.

Die Anmelderin hat aber darüber hinaus gefunden, dass Carnosine insbesondere die Aktivität der G6PDH Produktion steigert und der Apoptosis entgegen wirkt. Die Verwendung der Stoffe als schützende und aufbauende Medikamente oder Pflegemittel ist prinzipiell für alle Zubereitungen möglich, die zur Prävention gegen Schädigungen oder bei Schädigungen der Haut und damit in der Hautpflege und Prophylaxe eingesetzt werden. Eine andere Verwendung auf diesem Gebiet ist die Applikation bei empfindlicher, durch Allergie oder anderen Ursachen geschädigter Haut. Die Schädigung der Haut kann dabei unterschiedlichste Ursachen haben.

Die übliche Einsatzmenge der Carnosine beträgt bezogen auf die Emulsion typischerweise etwa 0,001 bis etwa 2,5 Gew.-%, vorzugsweise etwa 0,005 bis 2 Gew.-% und insbesondere etwa 0,01 bis etwa 1,5 Gew.-%.

### EMULGATOREN

Als Emulgatoren für die Emulsionen kommen beispielsweise nicht-ionogene Tenside aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEG-alkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich oder Cosmedia^{®} SP von Cognis;
- Polyalkylenglycole sowie
- Glycerincarbonat.

Im Folgenden werden besonders geeignete Emulgatoren näher erläutert:

**Alkoxylate.** Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

**Alkyl- und/oder Alkenyloligoglykosid.** Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

**Partialglyceride.** Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

**Sorbitanester.** Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

**Polyglycerinester.** Typische Beispiele für geeignete Polyglycerinester sind Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} Gl 34), Polyglyceryl-3 Oleate, Diisostearoyl Polyglyceryl-3 Diisostearate (Isolan^{®} PDI), Polyglyceryl-3 Methylglucose Distearate (Tego Care^{®} 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32) und Polyglyceryl Polyricinoleate (Admul^{®} WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

**Anionische Emulgatoren.** Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

**Amphotere und kationische Emulgatoren.** Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimi-dazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

Vielfach hat es sich als vorteilhaft erwiesen, Kombinationen unterschiedlicher Emulgatoren einzusetzen, nämlich binäre Mischungen (z.B. Alkylpolyglucosid/Fettalkoholethoxylat) oder ternäre Mischungen (z.B. Alkylpolyglucosid/Alkylbenzolsulfonat/Estequat oder Monoglycerid/Fettalkoholethoxylat/Fettalkohol).

Die Einsatzmenge der Emulgatoren kann zwischen etwa 1 und etwa 30 Gew.-% liegen. Bevorzugt sind Bereiche von etwa 2 bis 20 und insbesondere etwa 5 bis 10 Gew.-%. Entsprechend bevorzugte Kombinationen sind den Ausführungsbeispielen zu entnehmen.

### TRÄGER

Sowohl die Emulsionen können als optionale Komponente (d1) Träger bzw. Lösungsmittel enthalten, die ausgewählt sind aus der Gruppe, die gebildet wird von Wasser, Alkoholen, Estern, Butylenglycol, Dipropylenglycol, Ethanol, Ethoxydiglycol, Ethylacetat, Glycerin, Propanol, Isopropanol, Macrogole, Propylpropylenglycol(2)methylether, Propylpropyl-englycol(3)methylether, Propylencarbonat, Propylenglycol, Triethylenglycol, Isoparaffin, Amylacetat, Amylbenzoat, Benzylacetat, Butylacetat, Butylenglycol, Butyllactat, Butooctylbenzoat, Butooctylsalicylat, C10-C13 Alkane, C14-C17 Alkane, C11-C15 Cycloalkane, Caprylylbutyrat, Isoparaffine, Diacetin, Triacetin Dicaprylylether, Dicaprylylmaleat, und deren Mischungen.

Der Anteil der Träger an der Emulsion kann bis zu 50 Gew.-% ausmachen und liegt üblicherweise im Bereich von etwa 5 bis etwa 25 Gew.-% und insbesondere etwa 10 bis etwa 15 Gew.-%.

### ÖLKÖRPER

Weitergin können die Emulsionen Ölkörper (Komponente d2) aufweisen Hierfür kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, E-rucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhy-droxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol^{®} CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv^{®} TN), lineare oder verzweigte, symmetrische oder unsymmetrische Dialkylether mit 6 bis 22 Kohlenstoffatomen pro Alkylgruppe, wie z.B. Dicaprylyl Ether (Cetiol^{®} OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

Der Anteil der Ölkörper an der Emulsion kann bis zu 50 Gew.-% ausmachen und liegt üblicherweise im Bereich von etwa 5 bis etwa 25 Gew.-% und insbesondere etwa 10 bis etwa 15 Gew.

### WEITERE HILFS- UND ZUSATZSTOFFE

Die erfindungsgemäßen Emulsionen können weitere typische Hilfs- und Zusatzstoffe aufweisen, wie beispielsweise milde Tenside, Perlglanzwachse, Konsistenzgeber, Verdickungsmittel, Überfettungsmittel, Stabilisatoren, Polymere, Siliconverbindungen, Fette, Wachse, Lecithine, Phospholipide, UV-Lichtschutzfaktoren, Feuchthaltemittel, biogene Wirkstoffe, Antioxidantien, Deodorantien, Antitranspirantien, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. zwitterionische Tenside enthalten sein; die Beschreibung dieser Stoffe überlappt mit der für die Emulgatoren, da beide über oberflächenaktive Eigenschaften verfügen.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Fette und Wachse

Typische Beispiele für Fette sind Glyceride, d.h. feste oder flüssige pflanzliche oder tierische Produkte, die im Wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen

### Kühlstoffe

Kühlstoffe sind Verbindungen, die auf der Haut ein Gefühlt der Kälte erzeugen. In der Regel handelt es sich dabei um Mentholverbindungen, die - neben dem Grundkörper Menthol selber - beispielsweise ausgewählt aus der Gruppe, die gebildet wird von Menthol Methyl Ether, Menthone Glyceryl Acetal (FEMA GRAS¹ 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutamate (FEMA
¹ FEMA steht für "Flavor and Extracts Manufacturers Association" und GRAS ist definiert als "Generally Regarded As Safe". Eine FEMA GRAS Bezeichnung bedeutet, dass die so gekennzeichnete Substanz nach Standardmethode getestet und für toxikologisch unbedenklich erachtet wird. GRAS 4006), Menthoxy-1,2-propanediol (FEMA GRAS 3784), Menthoxy-2-methyl-1,2-propandiol (FEMA GRAS 3849) sowie den Menthancarbonsäureestern und -amiden WS-3, WS-4, WS-5, WS-12, WS-14 und WS-30 sowie deren Gemischen.

Ein erster wichtiger Vertreter dieser Stoffe stellt das Monomenthyl Succinate (FEMA GRAS 3810) dar. Sowohl das Succinat als auch das analoge Monomenthyl Glutarate (FEMA GRAS 4006) stellen wichtige Vertreter von Monomenthylestern auf Basis von Di- und Polycarbonsäuren dar:

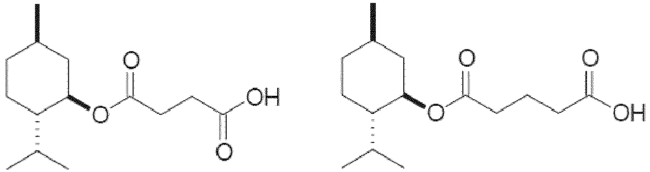

Beispiele für Anwendungen dieser Stoffe finden sich beispielsweise in den Druckschriften WO 2003 043431 (Unilever) oder EP 1332772 A1 (IFF).

Die nächste wichtige Gruppe von im Sinne der Erfindung bevorzugten Mentholverbindungen umfasst Carbonatester von Menthol und Polyolen, wie beispielsweise Glykolen, Glycerin oder Kohlenhydraten, wie beispielsweise Menthol Ethylenglycol Carbonate (FEMA GRAS 3805 = Frescolat^{®} MGC), Menthol Propylenglycol Carbonate (FEMA GRAS 3784 = Frescolat^{®} MPC), Menthol 2-Methyl-1,2-propandiol Carbonate (FEMA GRAS 3849) oder den entsprechenden Zuckerderivaten. Ebenfalls bevorzugt sind die Mentholverbindungen Menthyl Lactate (FEMA GRAS 3748 = Frescolat^{®} ML) und insbesondere das Menthone Glyceryl Acetal (FEMA GRAS 3807) bzw. Menthone Glyceryl Ketal (FEMA GRAS 3808), das unter der Bezeichnung Frescolat^{®} MGA vermarktet wird. Als ganz besonders vorteilhaft haben sich unter diesen Stoffen Menthone Glyceryl Acetal/Ketal sowie das Menthyl Lactate sowie Menthol Ethylene Glycol Carbonate bzw. Menthol Propylene Glycol Carbonat erwiesen, die die Anmelderin unter den Bezeichnungen Frescolat^{®} MGA, Frescolat^{®} ML, Frecolat^{®} MGC und Frescolat^{®} MPC vertreibt.

In den 70er Jahren des vergangenen Jahrhunderts wurden erstmals Mentholverbindungen entwickelt, die in der 3-Stellung über eine C-C-Bindung verfügen und von denen ebenfalls eine Reihe von Vertretern eingesetzt werden können. Diese Stoffe werden im Allgemeinen als WS-Typen bezeichnet. Grundkörper ist ein Mentholderivat, bei dem die Hydroxyl- gegen eine Carboxylgruppe ersetzt ist (WS-1). Von dieser Struktur leiten sich alle weiteren WS-Typen ab, wie beispielsweise die bevorzugten Spezies WS-3, WS-4, WS-5, WS-12, WS-14 und WS-30.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole^{®} und Pemulen-Typen von Goodrich; Synthalene^{®} von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone^{®} Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel und Stabilisatoren

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Stabilisatoren können Metallsalze von Fettsäuren, wie z.B. Magnesium-, Aluminium- und/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400^{®} von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat^{®} (BASF), Kondensationsprodukte von Polyglycolen und Aminen, quaternierte Kollagenpolypeptide, wie beispielsweise Lauryldimonium Hydroxypropyl Hydrolyzed Collagen (Lamequat^{®}L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine^{®}/Sandoz), Copolymere der Acrylsäure mit Dimethyl-diallylammoniumchlorid (Merquat^{®} 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar^{®} CBS, Jaguar^{®} C-17, Jaguar^{®} C-16 der Firma Celanese, quaternierte Ammoniumsalz-Polymere, wie z.B. Mirapol^{®} A-15, Mirapol^{®} AD-1, Mirapol^{®} AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäurean-hydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmeth-acrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

### Silikonverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### Feuchthaltemittel

Feuchthaltemittel dienen zur weiteren Optimierung der sensorischen Eigenschaften der Zusammensetzung sowie zur Feuchtigkeitsregulierung der Haut. Gleichzeitig wird die Kältestabilität der erfindungsgemäßen Zubereitungen, insbesondere im Falle von Emulsionen, erhöht. Die Feuchthaltemittel sind üblicherweise in einer Menge von 0,1 bis 15 Gew.-%, vorzugsweise 1 bis 10 Gew.-%, und insbesondere 5 bis 10 Gew.-% enthalten.

Erfindungsgemäß geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Erfindungsgemäß bevorzugt geeignet als Feuchthaltemittel sind Glycerin, Diglycerin, Triglycerin und Butylenglycol.

### Biogene Wirkstoffe und Antioxidantien

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. VitaminE-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

**Keimhemmende Mittel.** Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4-dichlorphenyl)harnstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

**Enzyminhibitoren.** Als Enzyminhibitoren sind beispielsweise Esteraseinhibitoren geeignet. Hierbei handelt es sich vorzugsweise um Trialkylcitrate wie Trimethylcitrat, Tripropy-lcitrat, Triisopropylcitrat, Tributylcitrat und insbesondere Triethylcitrat (Hydagen^{®} CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

**Geruchsabsorber.** Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

**Antitranspirantien.** Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
- adstringierende Wirkstoffe,
- Ölkomponenten,
- nichtionische Emulgatoren,
- Coemulgatoren,
- Konsistenzgeber,
- Hilfsstoffe wie z. B. Verdicker oder Komplexierungsmittel und/oder
- nichtwässrige Lösungsmittel wie z. B. Ethanol, Propylenglykol und/oder Glycerin.
Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichloro-hydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
- entzündungshemmende, hautschützende oder wohlriechende ätherische Öle,
- synthetische hautschützende Wirkstoffe und/oder
- öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Insektenrepellentien

Als Insekten-Repellentien kommen N,N-Diethyl-m-toluamid, 1,2-Pentandiol oder Ethyl Butylacetylaminopropionate in Frage.

### Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfaci-ne^{®} bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden, wie sie beispielsweise in der Publikation **"**Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### EMULSIONEN

In einer bevorzugten Ausführungsform weisen die Emulsionen die folgende Zusammensetzung auf:
(a) etwa 0,1 bis etwa 20 Gew.-%, vorzugsweise etwa 1 bis etwa 15 Gew.-% und insbesondere etwa 2 bis etwa 10 Gew.-% UV Lichtschutzfaktoren,
(b) etwa 0,001 bis etwa 2,5 Gew.-%, vorzugsweise etwa 0,005 bis etwa 2 Gew.-% und insbesondere etwa 0,01 bis etwa 1,5 Gew.-% Carnosinverbindungen,
(c) etwa 1 bis etwa 30 Gew.-%, vorzugsweise etwa 2 bis etwa 25 und insbesondere etwa 5 bis etwa 10 Gew.-% Emulgatoren,
(d) 0 bis etwa 50 Gew.-%, vorzugsweise etwa 5 bis etwa 40 Gew.-% und insbesondere etwa 10 bis etwa 20 Gew,.-% Träger, sowie
(e) 0 bis etwa 50 Gew.-%, vorzugsweise etwa 5 bis etwa 40 Gew.-% und insbesondere etwa 10 bis etwa 20 Gew,.-% Ölkörper
mit der Maßgabe, dass sich die Mengenangaben mit Wasser sowie gegebenenfalls weiteren Hilfs- und Zusatzstoffen zu 100 Gew.-% ergänzen.

Besonders bevorzugt sind Emulsionen, die die folgende Zusammensetzung aufweisen:
(a) etwa 1 bis etwa 15 Gew.-% und vorzugsweise etwa 2 bis etwa 10 Gew.-% UV Lichtschutzfaktoren,
(b) etwa 0,005 bis etwa 2 Gew.-%, vorzugsweise etwa 0,01 bis etwa 1,5 Gew.-% und insbesondere etwa 0,05 bis etwa 0,1 Gew.-% Carnosinverbindungen,
(c) etwa 2 bis etwa 25 und vorzugsweise etwa 5 bis etwa 10 Gew.-% Emulgatoren,
(d) 5 bis etwa 50 Gew.-%, vorzugsweise etwa 5 bis etwa 40 Gew.-% und insbesondere etwa 10 bis etwa 20 Gew,.-% Träger, sowie
(e) 5 bis etwa 50 Gew.-%, vorzugsweise etwa 5 bis etwa 40 Gew.-% und insbesondere etwa 10 bis etwa 20 Gew,.-% Ölkörper
mit der Maßgabe, dass sich die Mengenangaben gegebenenfalls mit weiteren Hilfs- und Zusatzstoffen zu 100 Gew.-% ergänzen.

Bei den Mengenangaben ist zu berücksichtigen, dass sie sich an den Fachmann richten. Dieser würde weder Zusammensetzungen in Betracht ziehen, die sich nicht zu 100 Gew.-% addieren oder darüber hinausgehen.

Schließlich sei darauf hingewiesen, dass sich die Erfindung auf solche Emulsionen bezieht, die thermodynamisch stabil sind und sich spontan oder unter geringem Eintrag von mechanischer Energie bilden. Sie erstreckt sich jedoch ebenso auf Emulsionen, die die gleiche Tröpfchengrößenverteilung aufweisen und auf anderem Wege hergestellt worden sind, beispielsweise durch Hochdruckhomogenisierung. Die Messung des Tröpfchengrößendurchmessers kann nach den dem Fachmann bekannten Verfahren erfolgen. Beispiele hierfür umfassen lasergestützte Verfahren wie etwa Photonenkorrelationsspektroskopie, Laserdiffraktometrie, Laserzählverfahren oder Turbidimetrie

### GEWERBLICHE ANWENDBARKEIT

Ein weiterer Gegenstand der Erfindung betrifft ein Verfahren zur Herstellung von Sonnenschutzmitteln in Emulsionsform, umfassend die folgenden Schritte:
(i) Bereitstellen mindestens eines UV-Lichtschutzfaktors;
(ii) Bereitstellen mindestens einer Carnosinverbindung;
(iii) Bereitstellen mindesten eines Emulgators
(iv) Vermischen der Komponenten (a) und (b) gegebenenfalls unter Zugabe von Trägern, Ölkörpern und/oder weiteren kosmetischen Zusatzstoffen unter Ausbildung einer Emulsion.

Schließlich betrifft die Erfindung auch die Verwendung von Carnosinverbindungen der Formel (I) als Stabilisatoren für Emulsionen und insbesondere Emulsionen, wobei diese vorzugsweise in Mengen im Bereich von etwa 0,001 bis etwa 2,5 Gew.-% - bezogen auf die Emulsion - eingesetzt werden.

Sofern nicht bereits ausdrücklich darauf verwiesen, beziehen sich die vorstehend genannten Ausführungsformen und bevorzugten Bereichsangaben auch auf das beanspruchte Verfahren und die beanspruchte Verwendung. Eine Wiederholung erübrigt sich daher.

Im Folgenden wird die Erfindung an Hand von Ausführungsbeispielen nähe erläutert, ohne dass sie aber darauf eingeschränkt wäre. Im ersten Teil finden sich experimentelle Untersuchungen, die den bisher nicht bekannten Charakter der Carnosinverbindungen als Wirkstoffe zum Schutz der Haut gegen Umwelteinflüsse belegt. Im zweiten Teil wird gezeigt, dass Carnosine in der Lage sind Emulsionen, die UV-Lichtschutzfaktoren enthalten, auch bei hohen Temperaturen über längere Zeiten stabil zu halten. Im letzten Teil werden schließlich Formulierungsbeispiele gegeben.

### BEISPIELE

### CARNOSINE ALS WIRKSTOFFE GEGEN UMWELTSCHÄDIGUNG DER HAUT

### BEISPIELE 1 BIS 6, VERGLEICHSBEISPIELE V1 UND V2

### Wirksamkeit gegen Hautalterung

Hintergrund: Das Enzym Glucose-6-phosphat Dehydrogenase (G6PDH) katalysiert den ersten Schritt des sogenannten "Pentose-Wegs", bei dem ein wesentlicher DNA-Baustein, nämlich die Desoxyribose gebildet wird. Dabei wird Glucose-6-phosphat (G6P) mit Hilfe von G6PDH in 6-Phosphatogluconat (6PG) überführt. Gleichzeitig wird das dabei erforderliche Coenzym NADP wird zu NADPH2 reduziert, welches seinerseits eine Vielzahl von anderen biologischen Reaktion wie z.B. das Recycling von Glutathion oder die Lipidsynthese katalysieren kann. Reduziertes Glutathion schützt viele Enzyme, die über SH-Gruppen verfügen und Zellen vor oxidativem Stress, wie beispielsweise UV-Einwirkung. Damit ist der G6PDH-Gehalt ein wichtiger Parameter für den Zellschutz und die die Hauterneuerung.

Methode: Die G6PDH-Aktivität wurde in-vitro an menschlichen Fibroblasten nach der enzymatischen Methode von Okada bestimmt, der DNA-Gehalt nach dem Verfahren von Desaulniers. Die Ergebnisse sind in **Tabelle 1** zusammengefasst. Angegeben sind die Ergebnisse von jeweils 3 Messreihen mit Dreifachbestimmung in %-rel gegenüber einer Blindprobe.

**Tabelle 1**

| Stimul ierung der G6PDH-Aktivität (Angabe in %-rel.) | | | | | | |
|---|---|---|---|---|---|---|
| **Subs tanz** | | **Konz. % w/v** | **D NA** | | **G6 PDH** | |
| | | | nach 3 d | nach 6 d | nach 3 d | nach 6 d |
| | Blindprobe | 0 | 100 | 100 | 100 | 100 |
| V1 | Uvinul A | 0,001 | 97 | 93 | 88 | 81 |
| V2 | Octocrinyl | 0,001 | 95 | 90 | 89 | 85 |
| 1 | L-Carnosin | 0,001 | 111 | 120 | 105 | 109 |
| 2 | Carnicin^{∗}HCl | 0,001 | 106 | 118 | 104 | 109 |
| 3 | L-Carnosin + Uvinul A (1:1) | 0,001 | 119 | 137 | 115 | 120 |
| 4 | L-Carnosin + Octocrinyl (1:1) | 0,001 | 120 | 141 | 116 | 125 |
| 5 | L-Carnosin + Uvasorb HEB (1:5) | 0,001 | 122 | 138 | 114 | 121 |
| 6 | L-Carnosin + NeoHeliopan (1:5) | 0,001 | 125 | 140 | 110 | 112 |

Wie die Beispiele zeigen führt die Gabe von Carnosinen dazu, dass der sowohl die Menge an zellularer DNA als auch G6PDH in statistisch signifikanter Weise ansteigt. Dieser an sich schon unerwartete Effekt kann durch Kombination mit UV-Lichtschutzfiltern noch gesteigert werden, obwohl diese alleine überhaupt keinen Effekt zeigen.

### BEISPIELE 7 UND 8

### Inhibierung der Apoptose-Induktion

Hintergrund: Die Apoptose ist im Gegensatz zur Nekrose ein natürlicher gezielter Zelltod bestimmter unerwünschter oder geschädigter Zellen. Es handelt sich um einen aktiven Prozess der Zellen (Selbstmord auf Befehl). Speziell bei der Hautalterung kommt es durch einen Mangel an Wachstumsfaktoren in der Haut zu einer induzierten Apoptose der Hautzellen. Bei den durch Apoptose betroffenen Zellen wird durch das spezifische Enzym Endonuclease die nukleare DNA abgebaut und die DNA-Fragmente in das Cytoplasma geschleust.

Methode: Untersucht wurde die Fähigkeit von Carnosinen, die durch einen Mangel an Wachstumsfaktoren induzierte Apoptose in humanen Hautzellen zu verhindern. An humanen Fibroblasten und humanen Keratinocyten wurden diese Tests in vitro durchgeführt. Die humanen Zellen wurden in einem Nährmedium (DMEM = Dulbecco Minimum Essential Medium von der Firma Life Technologie Sarl) mit 10 % fötalem Kälberserum (von der Firma Dutcher) kultiviert. Diesem Nährmedium wurde Bromodesoxyuridin (BrdU) zugegeben, welches in die DNA eingebaut wurde und später zum Nachweis der DNA-Fragmente in dem Cytoplasma diente. Nach zwei Tagen Inkubationsdauer wurde das Nährmedium gegen Nährmedium (DMEM) ohne fötales Kälberserum ausgetauscht. Die zu testende Aktivsubstanz wurde zugegeben. Zum Vergleich wurde eine Zellprobe ohne zu testende Aktivsubstanz inkubiert.

Nach einer weiteren Inkubationszeit von einem oder zwei Tagen bei 37 °C wurden die Zellen durch Trypsination zurückgewonnen nach der Methode von Dunnebacke und Zitcer beschrieben in: Cell and tissue culture, Hrsg.: J. Paul, Churchill Livingstone, 1975, S. 226. Nach der Trypsinierung wurden die Zellen zentrifugiert und ausgezählt. Anschließend wurde der Gehalt an BrdU in DNA-Fragmenten aus dem Cytoplasma mit Hilfe des ELISA Tests (ELISA Kit der Firma Roche) ermittelt. Der Gehalt an BrdU ist ein Maß für die DNA-Fragmente, die aus dem Nukleus, dem Zellkern, in das Cytoplasma geschleust wurden. Die Ergebnisse wurden auf eine Millionen Zellen bezogen und in Prozent im Vergleich zur Kontrolle angegeben. Die Ergebnisse sind in der folgenden **Tabelle 2** zusammengefasst.

**Tabelle 2**

| Anzahl der Zellen und Gehalt an D NA-Fragmenten | | | | |
|---|---|---|---|---|
| **Subst anz** | | **Konz. [% w/v]** | **Zellzählung** | **Gehalt an DNA-Fragmenten** |
| | Kontrolle | 0 | 100 | 100 |
| 7 | L-Carnosin | 0,001 | 129 | 85 |
| 8 | Carnicin^{∗}HCl | 0,001 | 118 | 88 |

Die in vitro Beispiele zeigen, dass durch den Einsatz von Carnosinen die Apoptose in humanen Zellkulturen und der Gehalt an freien DNA-Fragmenten im Cytoplasma und damit der Grad an zerstörter DNA im Zellkern abnehmen.

### BEISPIELE 9 UND 10

### Stimulierung der Synthese von dermalen Makromolekülen (GAG)

Hintergrund: Das Ziel dieser Untersuchungen ist der Nachweis einer stimulierenden Aktivität von Carnosinen auf die Synthese von dermalen Makromolekülen an humanen Fibroblastenkulturen in vitro. Die Dermis ist aufgebaut aus Zellen (Fibroblasten und Mastzellen), Gewebebestandteilen (Kollagen und Elastin) und aus sogenannten Grundsubstanzen. Zu diesen Grundsubstanzen zählen beispielsweise Glykosaminoglykan (GAG), Hyaluronsäure, Chondroitinsulfat, Dermatansulfat und Glycoproteine. Durch die Hautalterung vermindert sich die intermolekulare Verfestigung und Elastizitaet der Dermis und dadurch die Straffheit der Haut. Ebenso wird die Zahl der vorhandenen Hautzellen, insbesondere der Fibroblasten im Laufe der Hautalterung reduziert. Die Kollagenfasern werden im Laufe der Zeit fragmentiert und es erhöht sich der Anteil von unlöslichen zu löslichen Kollagen. Die feinen dermalen elastischen Fasern vergröbern sich und werden zerstört. Die Synthese von GAG (Glycosaminoglycan) ist vermindert. All diese Prozesse tragen zur Hautalterung und deren Erscheinungsformen wie Falten und mangelnde Straffheit der Haut bei.

Methode: Die Messmethode basiert auf eine Anfärbung von Makromolekülen in einer Kultur humaner Fibroblasten, die mit Kollagen Typ I ein Kollagen-Gel oder Kollagen Gitterfasern aufbaut. Bestimmte Regionen dieser Fasern werden mit Hilfe von Anfärbereagenzien auf den Anteil an den genannten Makromolekülen quantifiziert.

Zu diesem Zweck vermischte man eine Suspension humaner Fibroblasten mit einer Lösung von Kollagen Typ I (1-2 mg/ml). Diese Mischung wurde in einem definiertem Nährmedium (DMEM = Dulbecco Minimum Essential Medium, Firma Life Technologie Sarl) mit 0,5- oder 2 Gew.- percent fötalem Kälberserum (FCS) bei 37 °C in einer 5 %igen CO₂-Atmosphaere in Petri-Schalen (5 ml pro Schale) unter Zusatz verschiedener Konzentrationen der zu untersuchenden Pflanzenextrakte über 14 Tage inkubiert.

Die Kinetik der Kollagen-Gel Konzentration wurde 2 bis 3 mal pro Woche durch Messung von zwei perpendikularen Durchmessern an jedem Kollagen-Gel mit einem Mikroskop mit Bildanalysesystem bestimmt. Die Größe der Fläche ist in der Tabelle 8 in cm² dargestellt. Nach 14-taegiger Inkubation wurde die Dichte des Kollagen-Gels durch eine Bildanalyse mit einer Lichtquelle aus sichtbarem Licht bestimmt indem unterschiedliche Graustufen vergleichend untersucht wurden. Es handelt sich um eine relative Bestimmung der Dichte (0=klar oder weiß und 1=schwarz), die mit keiner Einheit versehen werden kann.

Nach 7 und nach 14 Tagen Inkubationszeit wurden Biopsien (Gewebeproben) genommen und histologische Schnitte des Kollagen-Gels mit humanen Fibroblasten erhalten. Die Synthese von Makromolekülen wurde untersucht und durch die Anfärbung von Glycosaminoglycan mit PAS-Alcian blau z.B. von der Firma SIGMA nach der Periodic-Acid-Schiff-Methode (PAS), beschrieben in: Mowry RW, Anal. NY Adad. Sci. 106 Art 2, 402, 1963, quantifiziert. Die Beurteilung der Stimulierung der Synthese von Makromolekülen wurde direkt in der Umgebung von Fibroblasten durchgeführt. Diese Zone wird auch als "Perifibroblasten Fläche" bezeichnet.

Eine Quantifizierung der "Perifibroblasten" Sekretion bzw. der Sekretion von Fibroblasten in die Peripherie wurde durch ein Image-Analysator mittels eines Mikroskops durchgeführt. Detektiert wurden reaktive Strukturen in der "Perifibroblasten-Fläche" und die unterschiedlichen Graustufen vergleichend bestimmt. Dabei wurden die Werte der Graustufen unterteilt von 0 = weiß bis 255=schwarz. Diese Parameter sind direkt proportional zur Intensität der Synthese von Makromolekülen und damit zum GAG-Anteil der Fibroblasten.

In der folgenden **Tabelle 3** sind die Ergebnisse der Werte dieser Parameter dargestellt und direkt als repräsentative Werte für die Syntheseaktivität der Fibroblasten zu sehen. Der Anteil der GAGs wird als relativer Wert der Graustufe beschrieben.

**Tabelle 3**

| Gehalt an GAGs in Gewebepropen humaner Fibroblasten mit Kollagen | | | | | | |
|---|---|---|---|---|---|---|
| **Subst anz** | | **Fläche [cm2]** | | **Dichte** | **GAGs Anteil** | |
| | | **7 Tage** | **14 Tage** | **14 Tage** | **7 Tage** | **14 Tage** |
| | Kontrolle^{∗} | 7,6 | 4,4 | 0,35 | 12,0 | 10,1 |
| 9 | 0,5 Gew.-% FCS + 0,01 Gew.-% L-Carnosin | 10,1 | 6,0 | 0,25 | 12,1 | 15,1 |
| 10 | 0,5 Gew.-% FCS + 0,01 Gew.-% Carnicin^{∗}HCl | 9,9 | 5,8 | 0,25 | 11,8 | 13,3 |

| | | | | | | |
|---|---|---|---|---|---|---|
| ^{∗} 5 Gew.-% Fötales Kalbsserum (FCS) | | | | | | |

Aus den Ergebnissen der Bestimmung des Glycosaminoglycan-Anteils in Gewebeproben von Kollagen-Gel mit Fibroblasten, speziell in der perifibroblasten Fläche" lässt sich nach 14tägiger Inkubationszeit gegenüber dem Standard unter Zugabe von Carnosinen eine signifikante Erhöhung des GAG Anteils erkennen. Dies deutet in vitro auf eine regenerierende und revitalisierende Aktivität von Carnosinen an humanen Fibroblasten hin.

### BEISPIELE 11 UND 12

### Inhibierung der Elastase-Aktivität

Hintergrund: Elastase ist eine Protease, die bei entzündlichen Zuständen von den Fibroblasten ausgeschieden wird und für den Abbau von dermalen Makromolekülen, wie z.B. Kollagen und Elastin und damit für die Hautalterung mitverantwortlich ist.

Methode: Zur Untersuchung der Wirksamkeit des Pflanzenextraktes die Freisetzung von Elastase zu inhibieren wurde Pankreaselastase (eine Serin-Protease) untersucht und als Substrat Elastin mit einem chromogenen synthetischen Substrat markiert. Das System wurde mit den Wirkstoffen über 30 min bei Raumtemperatur inkubiert und anschließend nach Zentrifugation die optische Dichte des Farbstoffes bei 410 nm bestimmt. Die Einsatzmenge der Extrakte betrug 0,3 Gew.- percent. Die Ergebnisse sind in **Tabelle 4** zusammengefasst. Die Angabe erfolgte relativ zu einer Kontrolle als Standard (= 0 %), als Standard diente a1-Antitrypsin.

**Tabelle 4**

| | Elastaseinhibierung | |
|---|---|---|
| **Substanz** | | **Inhibierung [%]** |
| 11 | L-Carnosin | 32 |
| 12 | Carnicin^{∗}HCl | 28 |

Die Ergebnisse verdeutlichen, dass Carnosine in der Lage sind, die Elastase und speziell die Pankreas-Elastase zu inhibieren.

### BEISPIELE 13 UND 14

### Inhibierung der Glycation von Kollagen.

Zum Nachweis, dass Carnosine die nicht-enzymatische Glycation von Makromolekülen inhibieren, wurde Kollagen des Typs I mit Glucose und den Extrakten über einen Zeitraum von 21 Tagen bei 45 °C behandelt. Anschließend wurden die Suspensionen zentrifugiert und der Gehalt an Schiffschen Basen in der überstehenden Flüssigkeit durch Fluoreszenzmessung bei 430 nm bestimmt. Die Ergebnisse sind in **Tabelle 5** zusammengefasst. Die Angaben beziehen sich wieder auf die Kontrolle als Standards (ohne Extrakt und ohne Glucose).

**Tabelle 5**

| Inhibieru ng der Glycation von Kollagen | | |
|---|---|---|
| **Substa nz** | | **Inhibierung [%]** |
| | Kontrolle ohne Glucose | 46 |
| | Kontrolle mit Glucose | 100 |
| 13 | L-Carnosin | 68 |
| 14 | Carnicin^{∗}HCl | 65 |

Die in vitro Ergebnisse weisen darauf hin, dass Carnosine in der Lage sind, die Glycation von Kollagen zu inhibieren und damit den Alterungsprozess der Dermis durch Glycation der Kollagenfasern zu verlangsamen.

### CARNOSINE ALS STABILISATOREN VON EMULSIONEN

### BEISPIELE 15 BIS 17, VERGLEICHSBEISPIELE V3 BIS V5 Stabilitätsuntersuchungen

Verschiedene Emulsionen enthaltend unterschiedliche UV-Lichtschutzfaktoren wurden unter Hochdruck homogenisiert, so dass der D50-Wert im Bereich von 0,1 bis 100 µm lag. Anschließend wurden sie unter Luftausschluss in 150 ml Deckelgläser abgefüllt und anschließend mit und ohne Zugabe von 0,2 Gew.-% Carnosin über einen Zeitraum von 6 Wochen im Trockenschrank bei 40 °C gelagert. Die Beurteilung erfolgte nach jeweils 1 Woche Lagerung gemäß folgender Skala: (++) = keine Veränderung; (+) = geringe Trübung; (#) = deutliche Trübung; (-) = getrennt.

Die Ergebnisse sind in **Tabelle 6** zusammengefasst. Die Beispiele 15 bis 17 sind erfindungsgemäß, die Beispiele V3 bis V5 dienen zum Vergleich.

**Tabelle 6**

| | | | | | | |
|---|---|---|---|---|---|---|
| | **15** | **V3** | **16** | **V4** | **17** | **V5** |
| Ethylhexyl salicylate | 5,0 | 5,0 | 5,0 | 5,0 | 2,0 | 2,0 |
| Homosalate | 2,0 | 2,0 | - | - | - | - |
| Ethylhexyl methoxycinnamate | - | - | 2,0 | 2,0 | 7,0 | 7,0 |
| Butyl methoxydibenzoylmethane | 2,0 | 2,0 | - | - | - | - |
| Homosalate | - | - | 2,0 | 2,0 | - | - |
| Phenylbenzimidazole Sulfonic Acid | - | - | - | - | 2,0 | 2,0 |
| Isoamyl-p-methoxycinnamate | 4,0 | 4,0 | - | - | - | - |
| Octocrylene | - | - | 6,0 | 6,0 | 6,0 | 6,0 |
| 4-Methylbenzylidenecamphor | - | - | 2,0 | 2,0 | - | - |
| Benzophenon-3 | - | - | - | - | 2,0 | |
| Carnosin | 0,2 | - | 0,2 | - | 0,2 | - |
| Polyglyceryl-2 dipolyhydroxystearate | 3,0 | 3,0 | 3,0 | 3,0 | 3,0 | 3,0 |
| Glyceryl oleate | 2,0 | 2,0 | 2,0 | 2,0 | 2,0 | 2,0 |
| Cetearyl isononanoate | 5,0 | 5,0 | 5,0 | 5,0 | 5,0 | 5,0 |
| Dicaprylyl ether | 5,0 | 5,0 | 5,0 | 5,0 | 5,0 | 5,0 |
| Glycerin | 5,0 | 5,0 | 5,0 | 5,0 | 5,0 | 5,0 |
| Wasser | Ad 100 | | | | | |

| **Zusammensetzung** | **15** | **V3** | **16** | **V4** | **17** | **V5** |
|---|---|---|---|---|---|---|
| ***Lagerstabilität*** | | | | | | |
| Nach 1 Woche | ++ | ++ | ++ | ++ | ++ | ++ |
| Nach 2 Wochen | ++ | ++ | ++ | ++ | ++ | ++ |
| Nach 3 Wochen | ++ | + | ++ | + | ++ | ++ |
| Nach 4 Wochen | ++ | + | ++ | # | ++ | + |
| Nach 5 Wochen | ++ | # | ++ | - | ++ | # |
| Nach 6 Wochen | ++ | - | + | - | + | - |

Die erfindungsgemäßen Emulsionen erweisen sich über 5 Wochen bei Lagerung bei 40 °c vollständig stabil; eine erste Eintrübung findet nach 6 Wochen statt. Zu diesem Zeitpunkt waren alle Vergleichsformulierungen ohne Carnosinzusatz bereits getrennt.

### FORMULIERUNGSBEISPIELE

### Sonnenschutzspray (Menge in Gew.-%)

| **Ingredients** | **INCI** | **Menge** |
|---|---|---|
| Water. demineralized | Water (aqua) | Ad 100 |
| Glycerol | Glycerol | 4.00 |
| 1.3 butylene glycol | Butylene glycol | 5.00 |
| Plantacare APG | Coco glycosides | 2.00 |
| D-Panthenol | Panthenol | 0.50 |
| Lara Care A-200 | Galactoarabinan | 0.25 |
| Baysilone oil M 10 | Dimethicone | 1.00 |
| Edeta BD | Disodium EDTA | 0.10 |
| Copherol 1250 | Tocopheryl acetate | 0.50 |
| Cetiol OE | Dicaprylyl ether | 3.00 |
| Neo Heliopan^{®} HMS | Homosalate | 5.00 |
| Neo Heliopan^{®} AV | Ethylhexyl methoxycinnamate | 6.00 |
| Neo Heliopan^{®} 357 | Butyl methoxydibenzoylmethane | 1.00 |
| Corapan TQ | Diethylhexylnaphthalate | 2.00 |
| Alpha Bisabolol | Bisabolol | 0.10 |
| Pemulen TR-2 | Acrylates/C10-30 alkyl acrylate crosspolymer | 0.25 |
| Carnosin | Carnosin | 0.01 |
| NaOH. 10% | Sodium hydroxide | 0.60 |
| Perfume oil | Fragrance | 0.20 |
| Phenoxyethanol | Phenoxyethanol | 0.40 |
| Solbrol M | Methylparaben | 0.10 |
| Solbrol P | Propylparaben | 0.10 |
| 4-hydroxyacetophenone | Hydroxyacetophenone | 0.50 |

### Sonnenschutzspray O/W. SPE 15-20 (Menge in Gew.-%)

| **Ingredients** | **INCI** | **Amount** |
|---|---|---|
| Dracorin^{®} GOC | Glyceryl Oleate Citrate. Caprylic/Capric Triglyceride | 2.00 |
| Corapan^{®} TQ | Diethylhexyl 2.6-Naphthalate | 3.00 |
| Neo Heliopan^{®} HMS | Homosalate | 7.00 |
| Neo Heliopan^{®} OS | Ethylhexyl Salicylate | 5.00 |
| Neo Heliopan^{®} 357 | Butyl Methoxydibenzoylmethane | 3.00 |
| Isoadipate | Diisopropyl Adipate | 6.00 |
| Baysilone^{®} Oil M10 | Dimethicone | 1.00 |
| Edeta^{®} BD | Disodium EDTA | 0.10 |
| Vitamin E Acetate | Tocopheryl Acetate | 0.50 |
| Carnosin | Carnosin | 0.05 |
| Dragosantol^{®} 100 | Bisabolol | 0.10 |
| Pemulen^{®} TR-2 | Acrylates/C10-30 Alkyl Acrylate Crosspolymer | 0.25 |
| Water | Water (Aqua) | Ad 100 |
| Glycerol 99.5 P. | Glycerol | 4.00 |
| Butylene Glycol | Butylene Glycol | 5.00 |
| Neo Heliopan^{®} Hydro (103089). used as 25% aq. solution neutralized with Biotive^{®} L-Arginine | Phenylbenzimidazole Sulfonic Acid | 8.00 |
| Biotive^{®} L-Arginine | Arginine | 0.55 |
| Perfume oil | Fragrance | 0.40 |
| Sobrol M | Methylparaben | 0.30 |
| 4-hydroxyacetophenone | Hydroxyacetophenone | 0.60 |

### Sonnenschutzcreme (W/O). SPF 40 (Menge in Gew.-%)

| **Ingredients** | **INCI** | **Amount** |
|---|---|---|
| Dehymuls PGPH | Polyglyceryl-2 dipolyhydroxystearate | 5.00 |
| Copherol 1250 | Tocopheryl acetate | 0.50 |
| Permulgin 3220 | Ozocerite | 0.50 |
| Zinc stearate | Zinc stearate | 0.50 |
| Tegosoft TN | C12-15 Alkyl benzoate | 10.00 |
| Neo Heliopan^{®} E1000 | Isoamyl-p-methoxycinnamate | 2.00 |
| Neo Heliopan^{®} 303 | Octocrylene | 5.00 |
| Neo Heliopan^{®} MBC | 4-Methylbenzylidene camphor | 3.00 |
| Zinc oxide. neutral | Zinc oxide | 5.00 |
| Water. distilled | Water (aqua) | Add 100 |
| EDTA BD | Disodium EDTA | 0.10 |
| Carnosin | Carnosin | 0.10 |
| Glycerol | Glycerol | 4.00 |
| Magnesium sulfate | Magnesium sulfate | 0.50 |
| Perfume oil | Parfum | 0.30 |
| Symdiol^{®} 68 | 1.2-Hexanediol. Caprylylglycol | 0.30 |

### Sonnenschutzmilch (W/O) (Menge in Gew.-%)

| **Ingredients** | **INCI** | **Amount** |
|---|---|---|
| Dehymuls PGPH | Polyglyceryl-2 dipolyhydroxystearate | 3.00 |
| Beeswax 8100 | Beeswax | 1.00 |
| Monomuls 90-0-18 | Glyceryl oleate | 1.00 |
| Zinc stearate | Zinc stearate | 1.00 |
| Cetiol SN | Cetearyl isononanoate | 5.00 |
| Cetiol OE | Dicaprylyl ether | 5.00 |
| Tegosoft TN | C12-15 alkyl benzoate | 4.00 |
| Vitamin E | Tocopherol | 0.50 |
| Neo Heliopan^{®} OS | Ethylhexyl salicylate | 5.00 |
| Neo Heliopan^{®} AV | Ethylhexyl methoxycinnamate | 7.50 |
| Uvinul^{®} T150 | Ethylhexyl triazone | 1.50 |
| Water. distilled | Water (Aqua) | To 100 |
| Trilon BD | Disodium EDTA | 0.10 |
| Glycerol | Glycerol | 5.00 |
| Neo Heliopan^{®} AP 10% solution. neutralized with NaOH | Disodium phenyl dibenzimidazole tetrasulfonate | 15.00 |
| Perfume oil | Parfum | 0.25 |
| Alpha bisabolol | Bisabolol | 0.10 |
| SymOcide^{®} PT | Phenoxyethanol. Tropolone | 0.25 |
| Carnosin | Carnosin | 0.10 |

## Patentansprüche

1. Verwendung von Carnosinverbindungen der Formel (I) 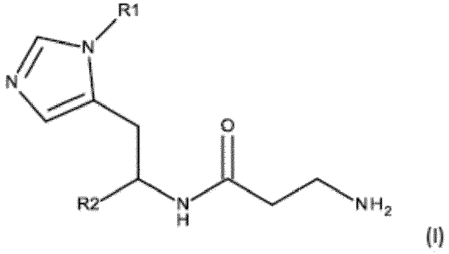 wobei R1 für H oder CH₃ und R2 für H oder COOH steht, oder deren Salze; als Stabilisatoren für Emulsionen.

2. Verwendung nach Anspruch 1, **dadurch gekennzeichnet, dass** man die Carnosinverbindungen in Mengen im Bereich von etwa 0,001 bis etwa 2,5 Gew.-% - bezogen auf die Emulsion - einsetzt.

3. Verwendung nach den Ansprüchen 1 und/oder 2, **dadurch gekennzeichnet, dass** die Emulsionen
(a) mindestens einen UV-Lichtschutzfaktor,
(b) mindestens eine Carnosinverbindung der Formel (I),
(c) mindestens einen Emulgator, sowie gegebenenfalls
(d1) Träger und/oder
(d2) Ölkörper
mit der Maßgabe enthalten, dass die Emulsionen eine Tröpfchengröße aufweisen, bei denen der D50-Wert im Bereich von 40 bis 100 µm liegt.

4. Verwendung nach Anspruch 3, **dadurch gekennzeichnet, dass** der D90-Wert im Bereich von 40 bis 150 µm liegt.

5. Verwendung nach den Ansprüchen 3 und/oder 4, **dadurch gekennzeichnet, dass** sowohl der D50- als auch der D90-Wert im Bereich von 40 bis 100 µm liegen.

6. Verwendung nach mindestens einem der Ansprüche 3 bis 5, **dadurch gekennzeichnet, dass** die UV-Lichtschutzfaktoren ausgewählt sind aus der Gruppe, die gebildet wird von organischen UV-A- und UV-B-Lichtschutzfaktoren.

7. Verwendung nach Anspruch 6, **dadurch gekennzeichnet, dass** die UV-A-Filter ausgewählt sind aus der Gruppe, die gebildet wird von Benzoylmethan- und Enaminverbindungen sowie deren Gemischen.

8. Verwendung nach Anspruch 6, **dadurch gekennzeichnet, dass** die UV-B-Filter ausgewählt sind aus der Gruppe, die gebildet wird von 3-(4-Methylbenzyliden)campher, 4-(Dimethylamino)benzoesäure-2-ethyl-hexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester, Salicylsäure-2-ethylhexylester, Salicylsäure-4-iso-propylbenzylester, Salicylsäurehomomenthylester, 2-Hydroxy-4-methoxybenzophenon, 2-Hydroxy-4-methoxy-4'-methylbenzophenon, 2,2'-Dihydroxy-4methoxybenzophenon, 4-Methoxybenzmalonsäuredi-2-ethyl-hexylester, 2,4,6-Trianilino-(p-carbo-2'-ethyl-1'-hexyloxy)-1,3,5-triazin, Octyl Triazon, Dioctyl Butamido Triazon, 1-(4-tert.Butylphenyl)-3-(4'methoxyphenyl)propan-1,3-dion, 2-Phenylbenzimidazol-5-sulfonsäure und deren Alkali-, Erdalkali-, Ammonium-, Alkylammonium-, Alkanolammonium- und Glucammoniumsalze, 1H-Benzimidazole-4,6-Disulfonic Acid, 2,2'-(1,4-Phenylene)Bis-, Disodium Salt, 2-Hydroxy-4-methoxybenzo-phenon-5-sulfonsäure und ihre Salze, 4-(2-Oxo-3-bornylidenme-thyl)benzolsulfonsäure und 2-Methyl-5-(2-oxo-3-bornyliden)sulfonsäure und deren Salze, sowie deren Gemischen.

9. Verwendung nach mindestens einem der Ansprüche 3 bis 8, **dadurch gekennzeichnet, dass** die Verbindung der Formel (I) ausgewählt ist aus der Gruppe die gebildet wird von Carnosin, L-Carnosin, D-Carnosin, D/L-Carnosin, Anserin, D-Anserin, L-Anserin sowie L-Anserin*HNO₃ und deren Mischungen.

10. Verwendung nach mindestens einem der Ansprüche 3 bis 9, **dadurch gekennzeichnet, dass** die Emulgatoren ausgewählt sind aus der Gruppe, die gebildet wird von Alkoxylaten, Alkyl- und/oder Alkenyloligoglykosiden, Partialglyceriden, Sorbitanestern, Polyglycerinestern, Fettsäuren, amphoteren und/oder kationischen Tensiden sowie deren Gemischen.

11. Verwendung nach mindestens einem der Ansprüche 3 bis 10, **dadurch gekennzeichnet, dass** die Träger ausgewählt sind aus der Gruppe, die gebildet wird von Wasser, Alkoholen, Estern, Butylenglycol, Dipropylenglycol, Ethanol, Ethoxydiglycol, Ethylacetat, Glycerin, Propanol, Isopropanol, Macrogole, Propylpropylengly- col(2)methylether, Propylpropylenglycol(3)methylether, Propylencarbonat, Propylenglycol, Triethylenglycol, Isoparaffin, Amylacetat, Amylbenzoat, Benzylacetat, Butylacetat, Butylenglycol, Butyllactat, Butooctylbenzoat, Butooctylsalicylat, C10-C13 Alkanen, C14-C17 Alkanen, C11-C15 Cycloalkanen, Caprylylbutyrat, Isoparaf-fin, Diacetin, Triacetin, Dicaprylylether und Dicaprylylmaleat sowie deren Gemischen.

12. Verwendung nach mindestens einem der Ansprüche 3 bis 11, **dadurch gekennzeichnet, dass** die Ölkörper ausgewählt sind aus der Gruppe, die gebildet wird von Guerbetalkohole auf Basis von Fettalkoholen mit 6 bis 18 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, Estern von linearen C₆-C₂₂-Fettsäuren mit verzweigten Alkoholen, Estern von C₁₈-C₃₈-Alkylhydroxycarbonsäuren mit linearen oder verzweigten C₆-C₂₂-Fettalkoholen, Estern von linearen und/oder verzweigten Fettsäuren mit mehrwertigen Alkoholen und/oder Guerbetalkoholen, Triglyceriden auf Basis C₆-C₁₀-Fettsäuren, flüssigen Mono-/Di-/Triglyceridmischungen auf Basis von C₆-C₁₈-Fettsäuren, Estern von C₆-C₂₂-Fettalkoholen und/oder Guerbetalkoholen mit aromatischen Carbonsäuren, Estern von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzlichen Ölen, verzweigten primären Alkoholen, substituierten Cyclohexanen, linearen und verzweigten C₆-C₂₂-Fettalkoholcarbonaten, Guerbetcarbonaten auf Basis von Fettalkoholen mit 6 bis 18, Estern der Benzoesäure mit linearen und/oder verzweigten C₆-C₂₂-Alkoholen, linearen oder verzweigten, symmetrischen oder unsymmetrischen Dialkylethern mit 6 bis 22 Kohlenstoffatomen pro Alkylgruppe, Ringöffnungsprodukten von epoxidierten Fettsäureestern mit Polyolen, Siliconölen, aliphatische bzw. naphthenische Kohlenwasserstoffen sowie deren Gemischen.

13. Verwendung nach mindestens einem der Ansprüche 1 bis 12, **dadurch gekennzeichnet, dass** die Emulsionen folgende Zusammensetzung aufweisen:
(a) 0,1 bis 20 Gew.-% UV Lichtschutzfaktoren,
(b) 0,001 bis 2,5 Gew.-% Carnosinverbindungen,
(c) 1 bis 30 Gew.-% Emulgatoren,
(d) 0 bis 50 Gew.-% Träger, sowie
(e) 0 bis 50 Gew.-% Ölkörper
mit der Maßgabe, dass sich die Mengenangaben mit Wasser sowie gegebenenfalls weiteren Hilfs- und Zusatzstoffen zu 100 Gew.-% ergänzen.

14. Verwendung nach Anspruch 13, **dadurch gekennzeichnet, dass** die Emulsionen folgende Zusammensetzung aufweisen:
(a) 0,1 bis 20 Gew.-% UV Lichtschutzfaktoren,
(b) 0,001 bis 2,5 Gew.-% Carnosinverbindungen,
(c) 1 bis 30 Gew.-% Emulgatoren,
(d) 5 bis 50 Gew.-% Träger, sowie
(e) 5 bis 50 Gew.-% Ölkörper
mit der Maßgabe, dass sich die Mengenangaben gegebenenfalls mit weiteren Hilfs- und Zusatzstoffen zu 100 Gew.-% ergänzen.

## Claims

1. The use of carnosine compounds corresponding to formula (I) 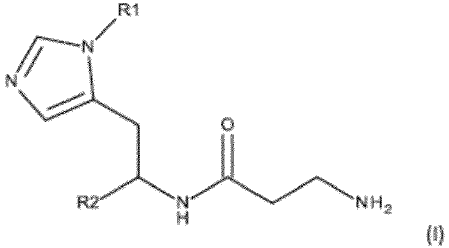 where R1 is H or CH3 and R2 is H or COOH, or salts thereof; as stabilizers for emulsions.

2. Use according to claim 1, **characterized in that** the carnosine compounds are used in amounts in the range from about 0.001 to about 2.5% by weight, based on the emulsion.

3. Use according to claims 1 and/or 2, **characterized in that** the emulsions contain
(a) at least one UV light protection factor,
(b) at least one carnosine compound of the formula (I),
(c) at least one emulsifier, and optionally
(d1) carriers and/or
(d2) oil bodies
with the proviso that the emulsions have a droplet size in which the D50 value is in the range from 40 to 100 µm.

4. Use according to claim 3, **characterized in that** the D90 value is in the range from 40 to 150 µm.

5. Use according to claims 3 and/or 4, **characterized in that** both the D50 and the D90 values lie in the range from 40 to 100 µm.

6. Use according to at least one of claims 3 to 5, **characterized in that** the UV light protection factors are selected from the group formed by organic UV-A and UV-B light protection factors.

7. Use according to claim 6, **characterized in that** the UV-A filters are selected from the group formed by benzoylmethane and enamine compounds and mixtures thereof.

8. Use according to claim 6, **characterized in that** the UV-B filters are selected from the group consisting of 3-(4-methylbenzylidene)camphor, 4-(dimethylamino)benzoic acid 2-ethyl-hexyl ester, 4-(dimethylamino)benzoic acid 2-octyl ester, 4-(dimethylamino)benzoic acid amyl ester; 4-methoxycinnamic acid-2-ethyl¬hexyl ester, 4-methoxycinnamic acid pro-pylester, 4-methoxycinnamic acid isoamyl ester 2-cyano-3,3-phenylcinnamic acid-2-ethylhexyl ester; Salicylic acid 2-ethylhexyl ester, salicylic acid 4-iso-propylbenzyl ester, salicylic acid homomentyl ester; 2-hydroxy-4-methoxybenzophenone, 2-hydroxy-4-me¬thoxy-4'-methylbenzophenone, 2,2'-dihydroxy-4methoxybenzophenone; 4-methoxybenzmalonic acid di-2-ethyl¬hexyl ester; 2,4,6-trianilino-(p-carbo-2'-ethyl-1'-hexyloxy)-1,3,5-triazine; octyl triazone; dioctyl buta-mido triazone; 1-(4-tert. Butylphenyl)-3-(4'methoxyphenyl)¬propane-1,3-dione; 2-phenylbenzimidazole-5-sulfonic acid and its alkali, alkaline earth, ammonium, al-alkylammonium, alkanolammonium and glucammonium salts; 1H-benzimidazole-4,6-disulfonic acid, 2,2'-(1,4-phenylene)bis-, disodium salt; 2-hydroxy-4-methoxybenzo-phenone-5-sulfonic acid and salts thereof; 4-(2-oxo-3-bornylideneme-thyl)benzenesulfonic acid and 2-methyl-5-(2-oxo-3-bornyli-dene)¬sulfonic acid and salts thereof and mixtures thereof.

9. Use according to at least one of Claims 3 to 8, **characterized in that** the compound of formula (I) is selected from the group consisting of carnosine, L-carnosine, D-carnosine, D/L-carnosine, anserine, D-anserine, L-anserine and L-anserine*HNO3 and mixtures thereof.

10. Use according to at least one of claims 3 to 9, **characterized in that** the emulsifiers are selected from the group consisting of alkoxylates, alkyl and/or alkenyl oligoglycosides, partial glycerides, sorbitan esters, polyglycerol esters, fatty acids, amphoteric and/or cationic surfactants and mixtures thereof.

11. Use according to at least one of claims 3 to 10, **characterized in that** the carriers are selected from the group consisting of water, alcohols, esters, butylene glycol, dipropylene glycol, ethanol, ethoxydiglycol, ethyl acetate, glycerol, propanol, isopropanol, macrogols, propylene glycol (2) methyl ether, propylene glycol (3) methyl ether, propylene carbonate, propylene glycol, triethylene glycol, isoparaffin, amyl acetate, amyl benzoate, benzyl acetate, butyl acetate, butylene glycol, butyl lactate, butooctyl benzoate, butooctyl salicylate, C10-C13 alkanes, C14-C17 alkanes, C11-C15 cycloalkanes, caprylyl butyrate, isoparaf-fin, diacetin, triacetin, dicaprylyl ether and dicaprylyl maleate, and mixtures thereof.

12. Use according to at least one of claims 3 to 11, **characterized in that** the oil bodies are selected from the group consisting of fatty alcohol-based Guerbet alcohols having 6 to 18 carbon atoms, esters of linear C6-C22 fatty acids with linear or branched C6-C22 fatty alcohols or esters of linear or branched C6-C22 fatty acids with linear or branched C6-C22 fatty alcohols. esters of branched C6-C13 carboxylic acids with linear or branched C6-C22 fatty alcohols, esters of linear C6-C22 fatty acids with branched alcohols, esters of C18-C38 alkyl hydroxycarboxylic acids with linear or branched C6-C22 fatty alcohols, esters of linear and/or branched fatty acids with polyhydric alcohols and/or Guerbet alcohols, triglycerides based on C6-C10 fatty acids, liquid mono-/di-/triglyceride mixtures based on C6-C18 fatty acids, esters of C6-C22 fatty alcohols and/or Guerbet alcohols with aromatic carboxylic acids, esters of C2-C12 dicarboxylic acids with linear or branched alcohols containing 1 to 22 carbon atoms or polyols containing 2 to 10 carbon atoms and 2 to 6 hydroxyl groups, vegetable oils, branched primary alcohols, substituted cyclohexanes, linear and branched C6-C22 fatty alcohol carbonates, Guer¬bet carbonates based on fatty alcohols with 6 to 18, esters of benzoic acid with linear and/or branched C6-C22 alcohols, linear or branched, symmetrical or asymmetrical dialkyl ethers with 6 to 22 carbon atoms per alkyl group, ring-opening products of epo-oxidized fatty acid esters with polyols, silicone oils, aliphatic or naphthenic hydrocarbons and mixtures thereof.

13. Use according to at least one of claims 1 to 12, **characterized in that** the emulsions have the following composition:
(a) 0.1 to 20% by weight UV light protection factors,
(b) 0.001 to 2.5% by weight of carnosine compounds,
(c) 1 to 30% by weight emulsifiers,
(d) 0 to 50% by weight of carriers, and
(e) 0 to 50% by weight of oil bodies
with the proviso that the quantities add up to 100% by weight with water and optionally other auxiliaries and additives.

14. use according to claim 13, **characterized in that** the emulsions have the following composition:
(a) 0.1 to 20% by weight UV light protection factors,
(b) 0.001 to 2.5% by weight carnosine compounds,
(c) 1 to 30% by weight emulsifiers,
(d) 5 to 50% by weight carriers, and
(e) 5 to 50% by weight of oil bodies
with the proviso that, if desired, the amounts add up to 100% by weight with further auxiliaries and additives.

## Revendications

1. Utilisation de composés de carnosine de formule (I) 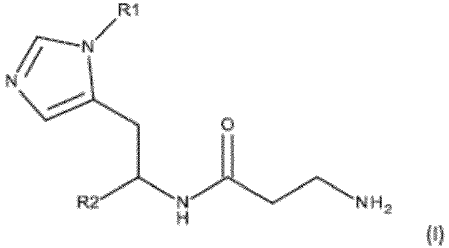 dans laquelle R1 représente H ou CH3 et R2 représente H ou COOH, ou leurs sels ; comme stabilisateurs pour des émulsions.

2. Utilisation selon la revendication 1, **caractérisée en ce que** l'on utilise les composés de carnosine en quantités de l'ordre d'environ 0,001 à environ 2,5 % en poids - par rapport à l'émulsion.

3. Utilisation selon les revendications 1 et/ou 2, **caractérisée en ce que** les émulsions sont
(a) au moins un UV facteur de protection solaire,
(b) au moins un composé de carnosine de formule (I),
(c) au moins un émulsifiant, ainsi qu'éventuellement
(d1) des supports et/ou
(d2) des huiles
à condition que les émulsions présentent une taille de gouttelettes pour lesquelles la valeur D50 se situe dans la plage de 40 à 100 µm.

4. Utilisation selon la revendication 3, **caractérisée en ce que** la valeur D90 se situe dans la plage de 40 à 150 µm.

5. Utilisation selon les revendications 3 et/ou 4, **caractérisée en ce que** les valeurs D50 et D90 sont toutes deux comprises dans la plage de 40 à 100 µm.

6. Utilisation selon au moins l'une des revendications 3 à 5, **caractérisée en ce que** les UV facteur de protection solaire, sont choisis dans le groupe formé par les UV-A et UV-B facteurs de protection solaire.

7. Utilisation selon la revendication 6, **caractérisée en ce que** les filtres UV-A sont choisis dans le groupe formé par les composés de benzoylméthane et d'énamine ainsi que leurs mélanges.

8. Utilisation selon la revendication 6, **caractérisée en ce que** les filtres UV-B sont choisis dans le groupe formé par le camphre de 3-(4-méthylbenzylidène), l'ester 2-éthylique de l'acide 4-(diméthylamino)benzoïque, l'ester 2-octylique de l'acide 4-(diméthylamino)benzoïque, l'ester amylique de l'acide 4-(diméthylamino)benzoïque ; Ester 2-éthylhexylique de l'acide 4-méthoxycinnamique, ester pro-pylique de l'acide 4-méthoxycinnamique, ester isoamylique de l'acide 4-méthoxycinnamique Ester 2-éthylhexylique de l'acide 2-cyano-3,3-phénylcinnamique ; 2-éthylhexyl ester de l'acide salicylique, 4-iso-propylbenzyl ester de l'acide salicylique, homomentyl ester de l'acide salicylique ; 2-hydroxy-4-méthoxybenzophénone, 2-hydroxy-4-me¬thoxy-4'-méthylbenzophénone, 2,2'-dihydroxy-4méthoxybenzophénone ; ester di-2-éthyl¬hexylique de l'acide 4-méthoxybenzmalonique ; 2,4,6-trianilino-(p-carbo-2'-éthyl-1'-hexyloxy)-1,3,5-triazine; octyl tria¬zone; dioctyl buta-mido triazone ; 1-(4-tert. butylphényl)-3-(4'méthoxyphényl)¬propane-1,3-dione; acide 2-phénylbenzimidazole-5-sulfonique et ses sels de métaux alcalins, alcalino-terreux, d'ammonium, d'alkylammonium, d'alcanolammonium et de glucammonium ; acide 1H-benzimidazole-4,6-disulfonique, 2,2'-(1,4-phénylène)bis-, sel de disodium ; l'acide 2-hydroxy-4-méthoxybenzo-phénone-5-sulfonique et ses sels ; l'acide 4-(2-oxo-3-bornylidèneméthyl)benzène-sulfonique et l'acide 2-méthyl-5-(2-oxo-3-bornylidène)-sulfonique et leurs sels ainsi que leurs mélanges.

9. Utilisation selon au moins l'une des revendications 3 à 8, **caractérisée en ce que** le composé de formule (I) est choisi dans le groupe formé par la carnosine, la L-carnosine, la D-carnosine, la D/L-carnosine, l'ansérine, la D-ansérine, la L-ansérine ainsi que la L-ansérine*HNO3 et leurs mélanges.

10. Utilisation selon au moins l'une des revendications 3 à 9, **caractérisée en ce que** les émulsifiants sont choisis dans le groupe formé par les alcoxylates, les alkyl- et/ou alcényloligoglycosides, les glycérides partielles, les esters de sorbitane, les esters de polyglycérol, les acides gras, les tensioactifs amphotères et/ou cationiques ainsi que leurs mélanges.

11. Utilisation selon au moins l'une des revendications 3 à 10, **caractérisée en ce que** les supports sont choisis dans le groupe formé par l'eau, les alcools, les esters, le butylèneglycol, le dipropylèneglycol, l'éthanol, l'éthoxydiglycol, l'acétate d'éthyle, la glycérine, le propanol, l'isopropanol, les macrogols, l'éther de propylpropylèneglycol(2)méthyle, l'éther de propylpropylèneglycol(3)méthyle, carbonate de propylène, propylenglycol, triéthylèneglycol, isoparaffine, acétate d'amyle, benzoate d'amyle, acétate de benzyle, acétate de butyle, butylèneglycol, lactate de butyle, benzoate de butooctyle, salicylate de butooctyle, alcanes en C10-C13, alcanes en C14-C17, cycloalcanes en C11-C15, butyrate de caprylyle, isoparaf-fin, diacétine, triacétine, éther de dicaprylyle et maléate de dicaprylyle, ainsi que leurs mélanges.

12. Utilisation selon au moins l'une des revendications 3 à 11, **caractérisée en ce que** les huiles sont choisis dans le groupe formé par les alcools de Guerbet à base d'alcools gras ayant de 6 à 18 atomes de carbone, les esters d'acides gras linéaires en C6-C22 avec des alcools gras linéaires ou ramifiés en C6-C22 ou les esters d'acides gras linéaires en C6-C22 avec des alcools gras ramifiés en C6-C22. esters d'acides carboxyliques en C6-C13 ramifiés avec des alcools gras en C6-C22 linéaires ou ramifiés, esters d'acides gras en C6-C22 linéaires avec des alcools ramifiés, esters d'acides alkyl-hydroxycarboxyliques en C18-C38 avec des alcools gras en C6-C22 linéaires ou ramifiés, Esters d'acides gras linéaires et/ou ramifiés avec des alcools polyvalents et/ou des alcools de Guerbet, triglycérides à base d'acides gras en C6-C10, mélanges liquides de mono-/di/triglycérides à base d'acides gras en C6-C18, esters d'alcools gras en C6-C22 et/ou d'alcools de Guerbet avec des acides carboxyliques aromatiques, Esters d'acides dicarboxyliques en C2-C12 avec des alcools linéaires ou ramifiés ayant de 1 à 22 atomes de carbone ou des polyols ayant de 2 à 10 atomes de carbone et de 2 à 6 groupes hydroxyle, des huiles végétales, des alcools primaires ramifiés, des cyclohexanes substitués, des alcoolcarbonates gras en C6-C22 linéaires et ramifiés, hydrocarbures naphténiques ainsi que leurs mélanges.

13. Utilisation selon au moins l'une des revendications 1 à 12, **caractérisée en ce que** les émulsions présentent la composition suivante :
(a) 0,1 à 20 % en poids de UV facteur de protection solaire,
(b) 0,001 à 2,5 % en poids de composés de carnosine,
(c) 1 à 30 % en poids d'émulsifiants,
(d) 0 à 50 % en poids de supports, et
(e) 0 à 50 % en poids de huiles
étant entendu que les données quantitatives se complètent à 100 % en poids avec de l'eau ainsi que, le cas échéant, d'autres adjuvants et additifs.

14. Utilisation selon la revendication 13, **caractérisée en ce que** les émulsions présentent la composition suivante :
(a) 0,1 à 20 % en poids de UV facteur de protection solaire,
(b) 0,001 à 2,5 % en poids de composés de carnosine,
(c) 1 à 30 % en poids d'émulsifiants,
(d) 5 à 50 % en poids de supports, et
(e) 5 à 50 % en poids de huiles
étant entendu que les données quantitatives se complètent éventuellement avec d'autres adjuvants et additifs à 100 % en poids.
